# EUROPEAN PATENT APPLICATION

(11) **EP 1 655 047 A1**
(43) Date of publication of application: **10.05.2006**
(21) Application number: 04029519.8
(22) Date of filing: 14.12.2004
(51) Int. Cl.: A61M 5/32

(54) **Protective structure for syringe**

(30) Priority: 09.11.2004 CN 200410092337
(71) Applicant: Chen, Chang-Tzu, Taipei (TW)
(72) Inventor: Chen, Chang-Tzu, Taipei (TW)
(74) Representative: Helms, Joachim

(57) **Abstract**

This invention discloses a syringe protective structure comprising a barrel (1) having a barrel base with an external diameter smaller than the external diameter of a barrel base; an opening with a size smaller than a barrel chamber at the front end of the barrel, a relay base (2) having an external diameter smaller than a base tube and being integrally extended to a tube rim with a larger external diameter and a smaller thickness; a front base pillar and a rear base pillar being disposed at the center and a base stair being formed between the base tube and the base rim; a bracket base (3) having a size corresponding to a base ring of the barrel base with a plurality of hooks bent (31) such that the relay base is disposed proximate to the opening of a barrel chamber, such that the liquid medication injection tube of the liquid medication cylinder is embedded into the rear base pillar hole, and the needle base is coupled to the front pillar base to define a liquid medication passage. After the injection is completed and withdrawn, the relay base together with the needle base are pulled. Therefore, the base rim is dropped under the hooks and the base stair is latched by the hooks into a fixed position.

## Description

### BACKGROUND OF THE INVENTION

### Field of the Invention

The present invention relates to a syringe protective structure, more particularly to a relay structure installed between an injection needle and a liquid medication injection cylinder, such that the injection needle cannot be removed without damaging the injection needle by external forces after an injection is completed and withdrawn, which serves as a protective structure for preventing any reuse of the syringe.

### Description of the Related Art

Diseases are inevitable to human beings and the rule of birth, age, illness and death governs the cycle of life of all living creatures. Of course, as the sanitary conditions are improved and the medicine is progressed, many diseases can be cured by appropriate medical treatments and medication by injection is a common indispensable part of the medical treatment.

On the other hand, the safety protection in a medial process should not be treated lightly; particularly the causes of many high-risk diseases come from blood infection. Therefore, medical professionals have to be very careful with the medication by injection, and if there is any mistake such as an accident of being pierced by the injection needle, the medical professional may be infected by unknown difficult-to-cure diseases (such as AIDS). Therefore, disposable injection needles or devices are popular in recent years, not only medical professional requiring this kind of needle, patients also having the same need. The protective structure for the disposable needles is developed and sold by manufacturers. Superstitious

There are many patented technologies related to the syringe protective structure, such as a syringe protective structure disclosed in the U.S. Pat. No. 5,338,310 entitled "Needle device having safety indication features", which comprises a barrel 2; a needle guard 32 being sheathed into the barrel 21, a recess being disposed on the internal wall of the needle guard, a collar 33 being sheathed into the barrel, and a projection being disposed corresponding to the collar 33 for being latched into the recess 37 after the injection is completed and withdrawn. In other words, with the joint operation of the barrel and the needle guard, the injection needle is not involved or the relay device is installed. Therefore, the inventor of the present invention believes that there must be some other solutions for the diversified products.

### Summary of the Invention

In view of the description above, the inventor of the present invention based on years of experience on the related industry to conduct extensive researches and experiments, and finally invented the syringe protective structure in accordance with the invention.

The primary objective of the present invention is to provide a syringe protective structure comprising a barrel having a barrel base with an external diameter smaller than the external diameter of a barrel base; an opening with a size smaller than a barrel chamber and being extended from the front end of the barrel to the barrel edge; a plurality of base latches being inwardly protruded from an end of the interior of the barrel base; a relay base having an external diameter smaller than a base tube and being integrally extended to a tube rim with a larger external diameter and a smaller thickness; a front base pillar and a rear base pillar being disposed at the center and having a front base pillar hole and a rear base pillar hole respectively, and a base stair being formed between the base tube and the base rim; a bracket base having a size corresponding to a base ring of the barrel base with a plurality of hooks bent and protruded from the top and a latch edge with a larger external diameter being disposed at the bottom and having a distance from the base edge corresponding to the height of the bracket base; such that the latch edge is latched by the base latches as to fix the barrel base, and the relay base is disposed proximate to the opening of a barrel chamber, such that the liquid medication injection tube of the liquid medication injection cylinder is embedded into the rear base pillar hole, and the needle base is coupled to the front pillar base to define a liquid medication passage. After the injection is completed and withdrawn, the relay base together with the needle base are pulled. Therefore, the base rim is dropped under the hooks and the base stair is latched by the hooks into a fixed position.

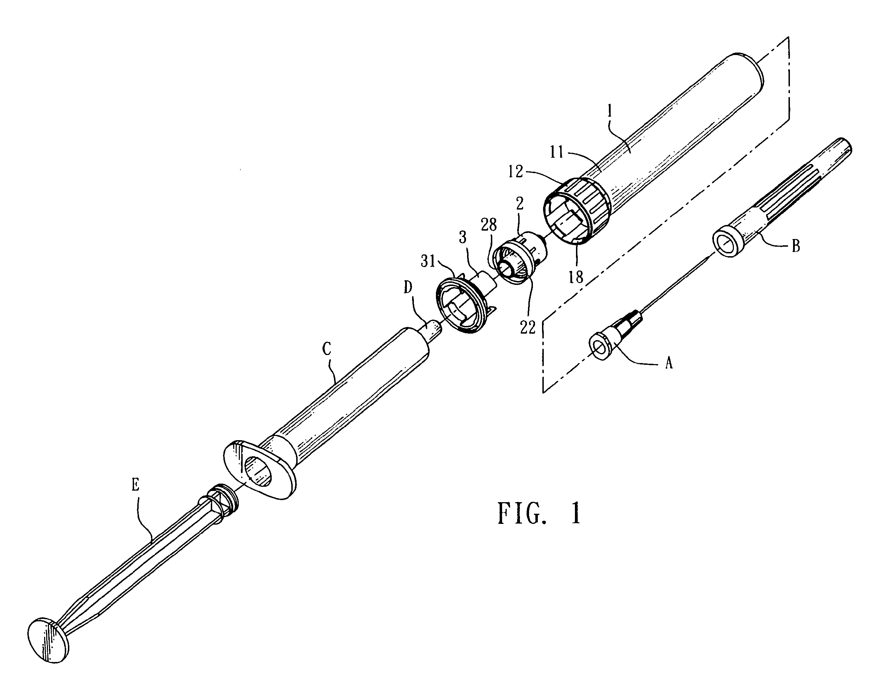

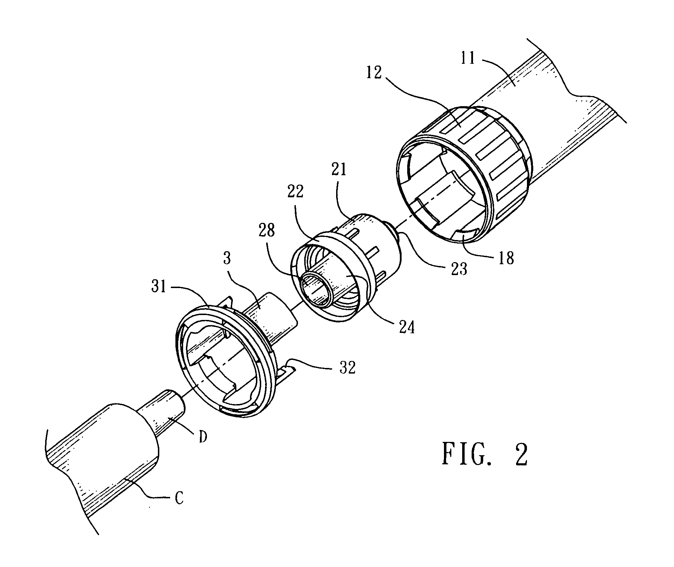

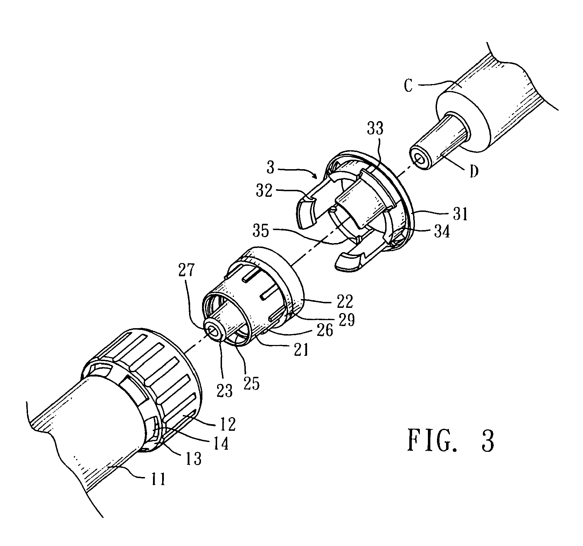

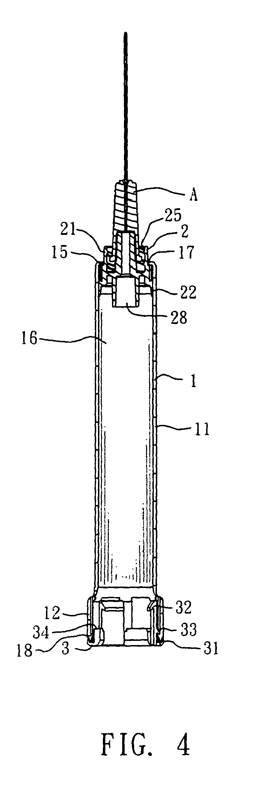

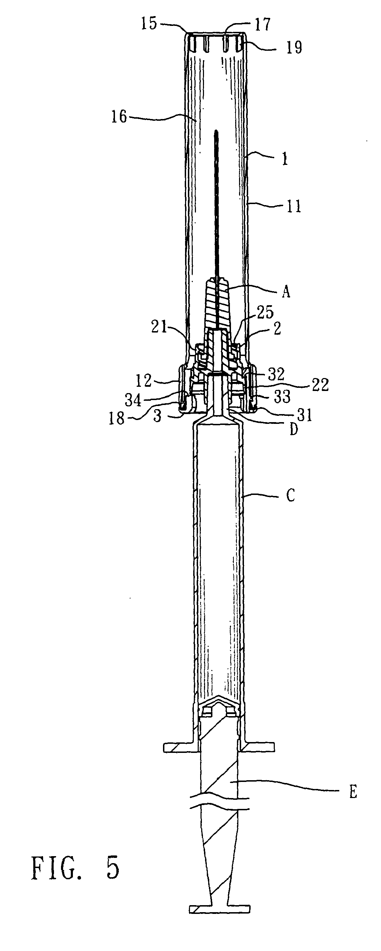

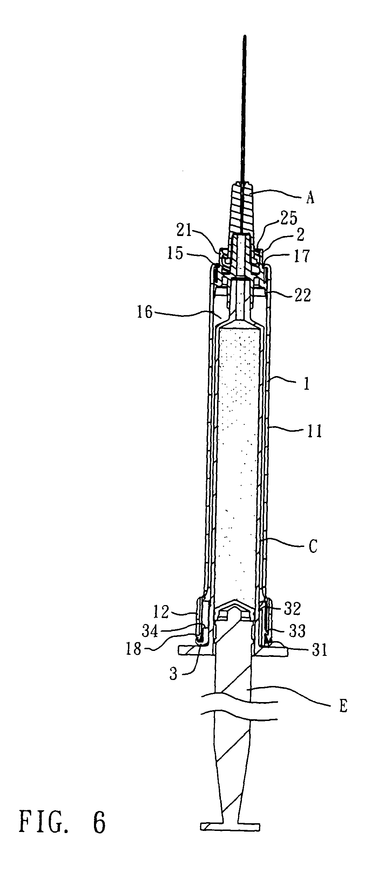

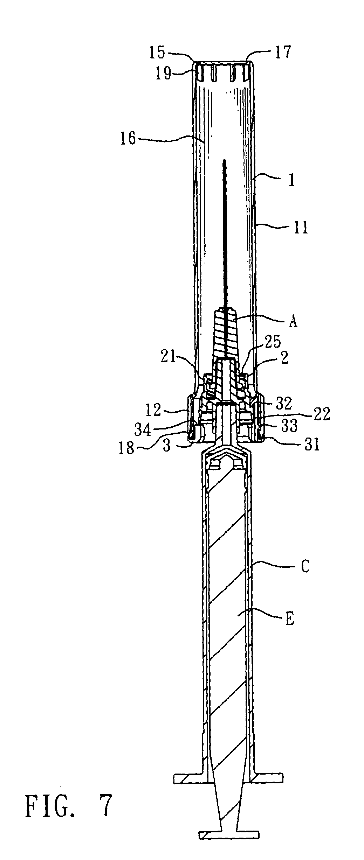

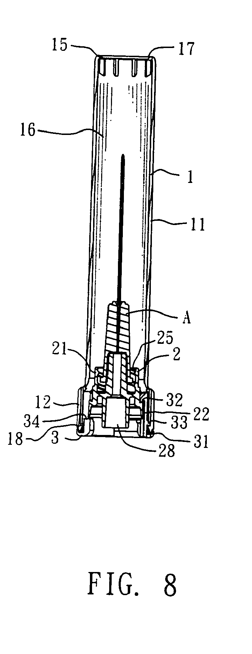

To further disclose the technical contents of the present invention, please refer to the figures, wherein FIG.1 is an exploded view of the syringe protective structure including the barrel of the present invention; FIG 2 is an enlarged view of some parts of FIG 1; FIG 3 is another enlarged view of some parts of FIG. 1 taken at other angle; FIG 4 is a sectional view of the assembled syringe structure according to the present invention; FIG 5 is a sectional view of the syringe structure including the barrel according to the present invention; FIG 6 is a sectional view of the syringe structure containing a liquid medication according to the present invention; FIG 7 is a sectional view of the syringe structure having its injection needle withdrawn to a fixed position after the injection is completed according to the present invention; and FIG 8 is a sectional view of the syringe structure with its barrel detached after the injection is completed according to the present invention.

### BRIEF DESCRIPTION OF THE DRAWINGS

To make it easier for our examiner to understand the objective of the invention, its structure, innovative features, and performance, we use a preferred embodiment together with the attached drawings for the detailed description of the invention.
FIG. 1 is an exploded view of the syringe protective structure including the barrel of the present invention.
FIG. 2 is an enlarged view of some parts of FIG 1.
FIG 3 is another enlarged view of some parts of FIG 1 taken at other angle.
FIG 4 is a sectional view of the assembled syringe structure according to the present invention.
FIG 5 is a sectional view of the syringe structure including the barrel according to the present invention.
FIG 6 is a sectional view of the syringe structure containing a liquid medication according to the present invention.
FIG 7 is a sectional view of the syringe structure having its injection needle withdrawn to a fixed position after the injection is completed according to the present invention.
FIG 8 is a sectional view of the syringe structure with its barrel detached after the injection is completed according to the present invention.
FIG 9 is the exploded view of showing the base tube providing the slots.

### DETAILED DESCRIPTION OF THE PREFERRED EMBODIMENTS

Please refer to the figures. A barrel 1 has a cylinder 11 with an external diameter smaller than the barrel 1 and the bottom of the cylinder 11 is coupled to a barrel base 12 with an external diameter larger than the barrel 1, and the cylinder 11 is coupled to the barrel 1 by a plurality of connecting plates 13 disposed on the periphery to define a through hole by the connecting holes 14 between every two adjacent connecting plates 13.

An opening 17 (as shown in FIG 4) with a diameter smaller than the diameter of a barrel chamber 16 inside is disposed at the front end of the cylinder 11, and integrally extended to the center of the barrel edge 15, and a plurality of curvedly protruded base latches 18 (as shown in FIG 2) is disposed inwardly at the ends of the interior of a barrel base 12, and a plurality of parallel reinforced ribs are disposed on the external wall of the barrel base 12. However, these components are prior arts, and thus will not be described here. Further, a plurality of parallel barrel ribs 19 is disposed on the internal wall of the opening 17 and serves a guide track of the relay base 2.

The relay base 2 comprises a base tube 21 having a smaller external diameter and an integrally extended base rim 22 having a larger external diameter and a smaller thickness with a good flexibility; a front base pillar 23 and a rear base pillar 24 both being disposed in the middle; wherein the interior of the base tube 21 has a thread and the external wall of the base tube 21 is surrounded with a plurality of base ribs 26, and an outwardly extended cross section with a smaller diameter is defined on both of the front and rear base pillars 23, 24, while each has a front base pillar hole 27 and a rear base pillar hole 28 being aligned in the same axial direction and inwardly tapered. Preferably, a drop of a base stair 29 is defined between the base tube 21 and the base rim 22. In actual implementation, the base rim 22 could be a plurality of rim brackets having a gap in between, and is not necessary to be the same as the one disclosed in the preferred embodiment of the present invention. However, this component is a prior art, and thus will not be described here.

The bracket base 3 is a base body with a size corresponding to the base ring 31 of the foregoing barrel base 12 and having a plurality of curvedly bent hooks 32 on the top surface and a latch edge 33 with a slightly larger external diameter disposed at the bottom proximate to the base ring 31, and a latch ring 34 with a smaller height being disposed between every two adjacent hooks 32, and an indention 35 is disposed on the top of both sides, and the distance between the latch edge 33 and the base ring 31 corresponds to the height of the base latch 18 for the latch after being sheathed.

Please refer to the figures for the assembly of the syringe protective structure of the present invention. The bracket base 3 is coupled to the barrel base 12, and the latch edge 33 falls into the base latch 18 of the barrel base 12 for the a fixed connection. In the meantime, the relay base 2 is disposed at the front of a barrel chamber 16 and proximate to an opening 17 and fixed by the base rib 26 and the barrel rib 19. A needle base A together with the injection needle is coupled to the base pillar 27 by the base hole, and is mounted into a fixed position in a fixed direction by the base thread 25 and screws. The assembled structure is shown in FIG 4. To prevent the accident of being pierced by the injection needle unintentionally, the needle base A can be sheathed by a sheath B. However, the sheath B is a prior art, and thus will not be described here.

Please refer to the figures. If the syringe protective structure of the present invention is used for injection, the liquid medication cylinder C is embedded into the barrel 1, and a liquid medication tube D disposed at the front end can be embedded into the base pillar hole 27 of the base tube 21 to constitute a close connection, and the push rod E can also be embedded into the liquid medication cylinder C as shown in FIG. 5.

In FIG 6, the push rod E is pulled outward to draw the liquid medication for the injection. Then, the liquid medication cylinder C is closely connected with the relay base 2. By pushing the push rod E, the liquid medication is ejected from the liquid medication tube D of the liquid mediation cylinder C to the injection needle.

Please refer to FIG 7. If the injection is completed and withdrawn, the liquid medication cylinder C together with the relay base are pulled backward, and the relay base 2 together with the needle base A are pulled backward accordingly. When they are pulled all the way to the bottom as shown in FIG 8, the base rim 22 together with the base stair 29 will drop between the hook 32 and the latch ring 34. The relay base 2 together with the needle base A will be stuck in the barrel A all the time, unless they are damaged by external forces. Such arrangement can prevent any reuse or the risk of being exposed.

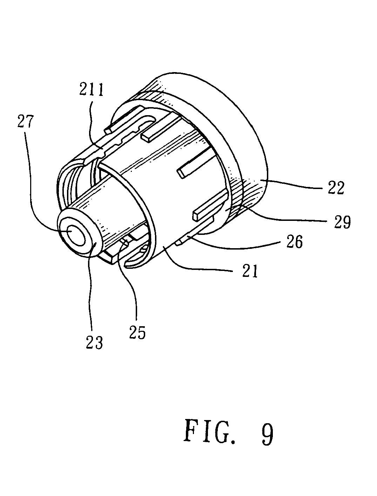

Refer to Fig. 9, in opposite position of said base ture 21, further provides two slots 211, the same effect can be achieved.

While the invention has been described by way of examples and in terms of preferred embodiments, it is to be understood that the invention is not limited thereto. To the contrary, it is intended to cover various modifications and similar arrangements and procedures, and the scope of the appended claims therefore should be accorded the broadest interpretation so as to encompass all such modifications and similar arrangements and procedures.

In summation of the above description, the present invention herein enhances the performance than the conventional structure and further complies with the patent application requirements and is submitted to the Patent and Trademark Office for review and granting of the commensurate patent rights.

## Claims

1. A syringe protective structure, comprising:
a barrel, having a cylinder with an external diameter smaller than the external diameter of a barrel base and being coupled to said barrel base and an opening with a size smaller than a barrel chamber and being extended from the front end of said barrel to said barrel edge and a plurality of base latches being inwardly protruded from an end of the interior of said barrel base;
a relay base, having an external diameter smaller than a base tube and being integrally extended to a tube rim with a larger external diameter and a smaller thickness; a front base pillar and a rear base pillar being disposed at the center and
having a front base pillar hole and a rear base pillar hole respectively, and a base stair being formed between said base tube and said base rim; and
a bracket base, having a size corresponding to a base ring of said barrel base with a plurality of hooks bent and protruded from the top and a latch edge with a larger
external diameter being disposed at the bottom and having a distance from said base edge corresponding to the height of said bracket base;
such that said latch edge is latched by said base latches as to fix said barrel base, and the relay base is disposed proximate to said opening of a barrel chamber, such that said liquid medication injection tube of said liquid medication injection cylinder is embedded into said rear base pillar hole, and said needle base is
coupled to said front pillar base to define a liquid medication passage; and after an injection is completed and withdrawn, said relay base together with said needle
base are pulled as to drop said base rim below said hooks and said base stair is latched by said hooks into a fixed position.

2. The syringe protective structure of claim 1, wherein said base latch has a concavely curved top surface.

3. The syringe protective structure of claim 1. wherein said opening comprises a barrel rib disposed on the internal wall of said opening, and a corresponding base rib disposed on the external wall of said base tube of said relay base.

4. The syringe protective structure of claim 1, wherein said base tube has a thread therein.

5. The syringe protective structure of claim 1, wherein said base rim comprises a plurality of gaps to define a plurality of rim brackets.

6. The syringe protective structure of claim 1, wherein said hook has a concavely curved top surface.

7. The syringe protective structure of claim 1, wherein said front base pillar and said rear base pillar are tapered aslant along the external axial direction and said front base pillar hole and said rear base pillar hole are tapered aslant towards the internal side.

8. The syringe protective structure of claim 1, wherein said cylinder and said barrel base are coupled by a plurality of connecting brackets having a connecting hole passing through.

9. The syringe protective structure of claim 1, wherein at least two slots can be provided in the opposite portion of said base tube.
